(19) Europäisches Patentamt
European Patent Office
Office européen des brevets

(11) Veröffentlichungsnummer: **0 004 904**
**B1**

(12) # EUROPÄISCHE PATENTSCHRIFT

(45) Veröffentlichungstag der Patentschrift: **29.07.81**

(21) Anmeldenummer: **79101018.4**

(22) Anmeldetag: **04.04.79**

(51) Int. Cl.$^3$: **C 07 D 239/70,**
C 07 D 401/12,
C 07 D 403/04,
C 07 D 403/12,
C 07 D 405/14,
C 07 D 417/04,
A 61 K 31/505

(54) **2-Amino-3a,4,5,6-tetrahydro-perimidin-Derivate, diese enthaltende Arzneimittel und Verfahren zu ihrer Herstellung.**

(30) Priorität: **14.04.78 DE 2816123**
**08.09.78 DE 2839137**

(43) Veröffentlichungstag der Anmeldung:
**31.10.79 Patentblatt 79/22**

(45) Bekanntmachung des Hinweises auf die Patenterteilung:
**29.07.81 Patentblatt 81/30**

(84) Benannte Vertragsstaaten:
**BE CH DE FR GB IT NL SE**

(56) Entgegenhaltungen:
**US - A - 3 502 647**
**US - A - 3 985 880**

**Chemical Abstracts. Band 81, Nr.5, 5.August 1974, Columbus, Ohio, USA**
**A.F. POZHARSKII et al. "Heterocyclic analogs of pleiadiene.XI.Synthesis of 1-(β-dialkylaminoalkyl)-and 2-aminoperimidines" Seite 430, Spalte 1, Abstract Nr.25625 t & Khim.**

**Chemical Abstracts, Band 86, Nr.17, 25.April 1977, Columbus, Ohio, USA**
**I.V. KOMISSAROV et al. "Synthesis and neurotropic activity of 2-aminoperimidines" Seite 544,Spalte 1, Abstracts Nr.121278f**

(73) Patentinhaber: **BAYER AG**
**Zentralbereich Patente, Marken und Lizenzen**
**D-5090 Leverkusen 1, Bayerwerk (DE)**

(72) Erfinder: **Kabbe, Hans-Joachim, Dr.**
**Walter-Flex Strasse 16**
**D-5090 Leverkusen 1 (DE)**
Erfinder: **Mayer, Karl Heinrich, Dr.**
**Bürgerbuschweg 100**
**D-5090 Leverkusen 3 (DE)**
Erfinder: **Ziemann, Heinz, Dr.**
**Am Wiesenberg 10**
**D-5653 Leichlingen 1 (DE)**
Erfinder: **Stoepel, Kurt, Dr.**
**In Den Birken 69**
**D-5600 Wuppertal 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 004 904 B1

2-Amino-3a,4,5,6-tetrahydro-perimidin-Derivate, diese enthaltende Arzneimittel und Verfahren zu ihrer Herstellung

Die vorliegende Erfindung betrifft neue 2-Amino-3a,4,5,6-tetrahydro-perimidin-Derivate, ein Verfahren zu ihrer Herstellung sowie ihre Verwendung in Arzneimittel insbesondere in kreislaufwirksamen Arzneimitteln.

Gegenstand der Anmeldung sind neue 2-Amino-3a,4,5,6-tetrahydro-perimidin-Derivate der tautomeren Formel (I)

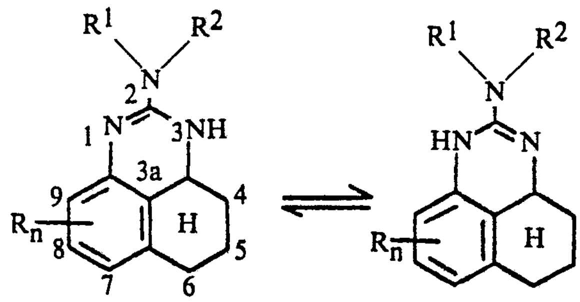

(Ia) (I) (Ib)

in welcher

R für Wasserstoff, Chlor oder Brom steht,

n 1 oder 2 bedeutet

$R^1$ für Wasserstoff, Alkyl mit 1 bis 4 Kohlenstoffatomen oder Phenyl steht und

$R^2$ für Wasserstoff, Phenyl, Acyl mit 2 bis 4 Kohlenstoffatomen, Alkyl mit 1 bis 4 Kohlenstoffatomen, wobei der Alkylrest gegebenenfalls substituiert ist durch Phenyl, Chlorphenyl, Pyridyl oder Piperazino, wobei der Piperazinorest gegebenenfalls substituiert ist durch Alkyl mit 1 bis 4 Kohlenstoffatomen, oder für Amino, Phenylamino, Pyridylamino, Mono- und Dialkylamino mit jeweils 1 bis 4 Kohlenstoffatomen in den Alkylgruppen steht oder

$R^1$ und $R^2$ gemeinsam für einen Alkylenrest mit bis zu 6 Kohlenstoffatomen stehen, wobei dieser Alkylenrest gegebenenfalls durch Sauerstoff, den $SO_2$— oder den NR″-Rest unterbrochen ist, wobei

R″ für Wasserstoff, Alkyl mit 1 bis 4 Kohlenstoffatomen, Benzyl, Phenyl — gegebenenfalls substituiert durch Halogen, Nitro, Amino oder Alkoxycarbonylamino mit 1 bis 4 Kohlenstoffatomen in der Alkoxygruppe — oder für die Gruppe

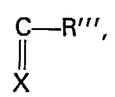

wobei

X Sauerstoff, Schwefel oder NH bedeutet, und

R‴ für Alkyl mit 1 bis 4 Kohlenstoffatomen, Alkoxy mit 1 bis 4 Kohlenstoffatomen, Furyl, Phenyl, Benzyl, Amino, Mono- und Dialkylamino mit jeweils 1 bis 4 Kohlenstoffatomen im Alkylrest, Phenylamino oder Pyridylamino steht,

sowie ihre pharmakologisch verträglichen Salze,

wobei sowohl die Verbindungen in ihrer racemischen Form als auch ihre optischen Antipoden zum Gegenstand dieser Anmeldung gehören.

Es wurde gefunden, daß man die neuen 2-Amino-3a,4,5,6-tetrahydro-perimidin-Derivate der Formel (I) erhält, wenn man 3a,4,5,6-Tetrahydro-perimidine der tautomeren Formel (II)

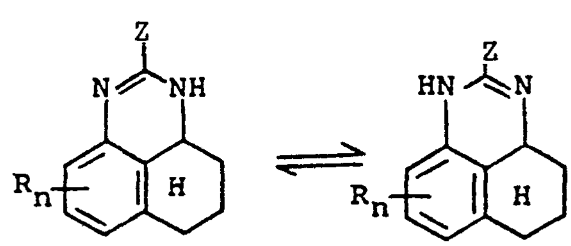

(IIa) (IIb)

in welcher

R und n die oben angegebene Bedeutung haben und

Z für eine nucleophil substituierbare Abgangsgruppe wie Halogen, Alkoxy, Aryloxy, Mercapto, Alkylmercapto, Arylmercapto, Alkylsulfonyl, Arylsulfonyl oder Sulfo steht, wobei die vorgenannten Alkyl- und Alkoxyreste jeweils 1 bis 8 Kohlenstoffatome enthalten, und Aryl für Phenyl oder Naphthyl steht,

mit Aminen der Formel

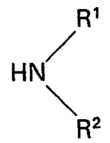

(III)

in welcher

$R^1$ und $R^2$ die oben angegebene Bedeutung haben,

oder mit Salzen dieser Amine mit anorganischen oder organischen Säuren in Gegenwart von inerten organischen Lösungsvermittlern und gegebenenfalls in Gegenwart von Säurebindern bei Temperaturen zwischen 20°C und 250°C umsetzt,

und gegebenenfalls Verbindungen der Formel (I), welche noch eine NH- oder $NH_2$-Gruppe besitzen, anschließend in an sich bekannter Weise acyliert, wobei eine $NH_2$-Gruppe auch zuvor durch Reduktion einer $NO_2$-Gruppe in an sich bekannter Weise erzeugt werden kann.

Zu dieser nachträglichen Acylierung eignen sich besonders Halogenide von Carbonsäuren und Sulfonsäuren sowie Kohlensäureesterhalogenide, Carbonsäureanhydride, Pyrokohlensäureester, Isocyanate, Senföle und Imidoester.

In dem folgenden Reaktionsschema werden einige Umsetzungs-beispiele exemplarisch gezeigt:

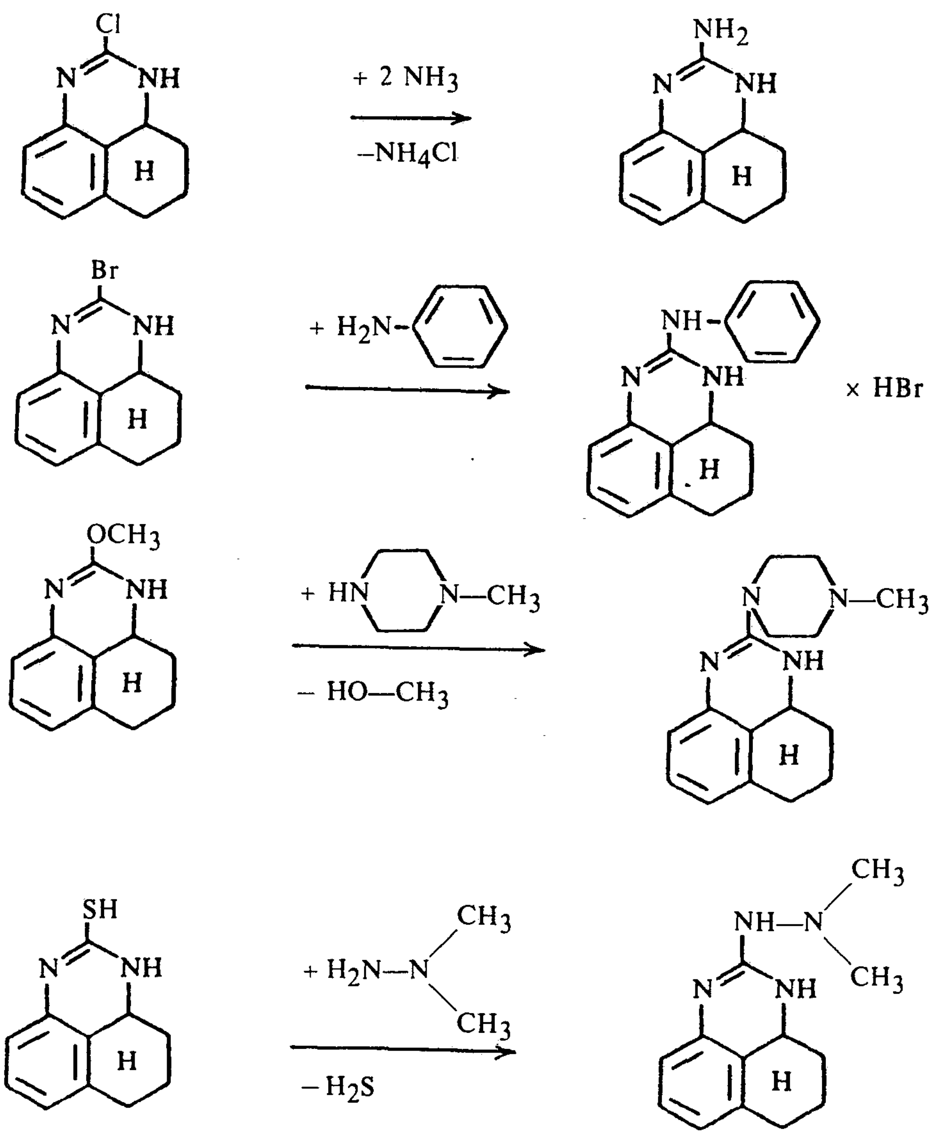

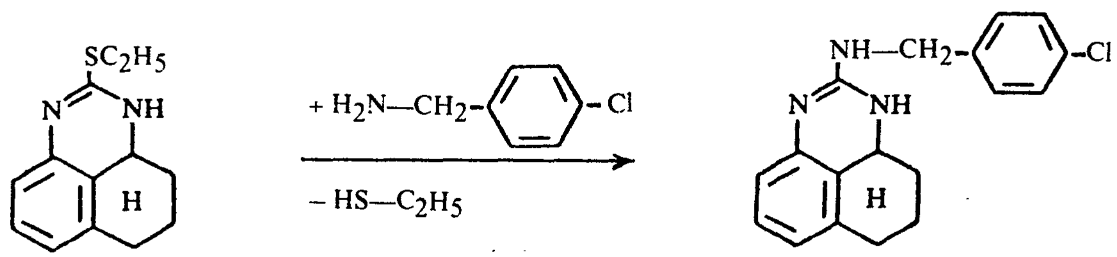


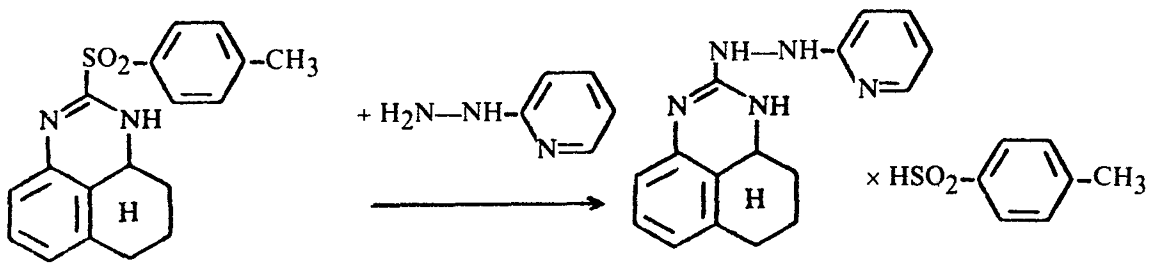

Beispiel für nachträgliche Acylierungen von Amino-funktionen:

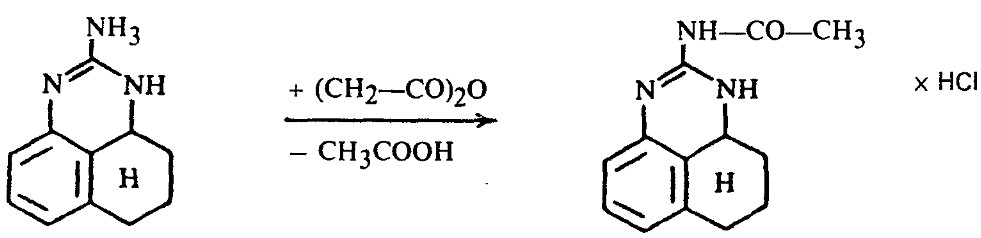


Zur Vereinfachung ist in den vorstehenden Reaktionsgleichungen jeweils nur die der tautomeren Formel (Ia) entsprechende Konstitution angegeben.

Die als Ausgangsstoffe zu verwendenden 3a,4,5,6-Tetrahydro-perimidine der tautomeren Formel (II) können z.B. hergestellt werden durch katalytische Hydrierung der bekannten 2-Hydroxy-perimidine und anschließende Halogenierung der 2-Position.

Beispielhaft für die Ausgangsverbindungen der Formel (II) seien genannt:

2-Chlor-3a,4,5,6-tetrahydro-perimidin
2-Brom-3a,4,5,6-tetrahydro-perimidin
2-Fluor-3a,4,5,6-tetrahydro-perimidin
2-Jod-3a,4,5,6-tetrahydro-perimidin
2-Methoxy-3a,4,5,6-tetrahydro-perimidin
2-Äthoxy-3a,4,5,6-tetrahydro-perimidin
2-Isopropoxy-3a,4,5,6-tetrahydro-perimidin
2-Butoxy-3a,4,5,6-tetrahydro-perimidin
2-Benzyloxy-3a,4,5,6-tetrahydro-perimidin
2-Phenyloxy-3a,4,5,6-tetrahydro-perimidin
2-Mercapto-3a,4,5,6-tetrahydro-perimidin
2-Methylmercapto-3a,4,5,6-tetrahydro-perimidin
2-Äthylmercapto-3a,4,5,6-tetrahydro-perimidin
2-Carboxymethylmercapto-3a,4,5,6-tetrahydro-perimidin
2-Benzylmercapto-3a,4,5,6-tetrahydro-perimidin
2-Phenylmercapto-3a,4,5,6-tetrahydro-perimidin
2-Methylsulfonyl-3a,4,5,6-tetrahydro-perimidin
2-Phenylsulfonyl-3a,4,5,6-tetrahydro-perimidin
2-(4-Methyl-phenyl)-sulfonyl-3a,4,5,6-tetrahydro-perimidin
2-Sulfo-3a,4,5,6-tetrahydro-perimidin
2,7-Dichlor-3a,4,5,6-tetrahydro-perimidin
2,9-Dichlor-3a,4,5,6-tetrahydro-perimidin
2-Chlor-7-brom-3a,4,5,6-tetrahydro-perimidin
2,7-Dibrom-3a,4,5,6-tetrahydro-perimidin
2,7,9-Trichlor-3a,4,5,6-tetrahydro-perimidin

2-Methoxy-7-chlor-3a,4,5,6-tetrahydro-perimidin
2-Mercapto-9-chlor-3a,4,5,6-tetrahydro-perimidin
2-Benzylmercapto-7-brom-3a,4,5,6-tetrahydro-perimidin

Die als Ausgangsstoffe zu verwendenden primären und sekundären Amine bzw. Hydrazine der Formel (III) sind bekannt oder lassen sich nach bekannten Verfahren herstellen.

Als Beispiele seien im einzelnen genannt:

Methylamin, Isobutylamin, 4-Chlorphenylethanamin, 4-Aminomethylpyridin, N-Aminoethyl-N'-methyl-piperazin, Dimethylamin, Morpholin, Piperazin, 4-Methylpiperazin, 4-Phenylpiperazin, 4-Fluorphenylpiperazin, 4-(2-Chlorphenyl)-piperazin, 4-(3,5-Dichlorphenyl)-piperazin, 4-Aminophenylpiperazin, 4-(Furyl-(2)-carbonyl)-piperazin, 4-(3-Chlorphenyl)-piperazin, 4-Ethoxy-carbonylpiperazin, Hexamethylenimin, Methylhydrazin, Phenylhydrazin, 2-Hydrazinopyridin, N,N-Dimethylhydrazin.

Als Lösungsvermittler kommen alle organischen Lösemittel in Frage, die gegenüber den jeweiligen Reaktionspartnern inert sind. Hierzu gehören vorzugsweise aliphatische Alkohole wie Methanol, Äthanol, Isopropanol oder Butanol, Äther wie Tetrahydrofuran, Dioxan, Äthylenglykolmonomethyläther, Äthylenglykoldiäthyläther, Glykole wie Äthylenglykol, Propylenglykol, Diäthylenglykol und entsprechende Äther mit aliphatischen Alkoholen wie Diäthylenglykoldimethyläther, Kohlenwasserstoffe wie Ligroin, Toluol, Xylol, Tetralin, Halogenkohlenwasserstoffe wie Chloroform, Tetrachlorkohlenstoff, Chlorbenzol, Dichlorbenzole, Nitrile wie Acetonitril, Propionitril, Carbonsäureamide wie Dimethylformamid, Dimethylacetamid, heterocyclische Basen wie Pyridin, Picoline, Kollidine, Chinolin oder Isochinolin, ferner handelsübliche technische Gemische dieser Lösemittel.

Die Umsetzung kann bei Normaldruck aber auch bei erhöhtem Druck durchgeführt werden. Insbesondere bei der Verwendung von Ammoniak oder niedrig siedenden primären Aminen als Reaktionspartner kann erhöhter Druck für die Umsetzung erforderlich sein.

Die Reaktionstemperaturen können in einem größeren Bereich variiert werden. Im allgemeinen arbeitet man bei Temperaturen zwischen 10 und 250°C, vorzugsweise zwischen 20 und 180°C, insbesondere zwischen 40 und 150°C.

Als Säurebindemittel können alle üblichen Säurebinder eingesetzt werden. Hierzu gehören anorganische Basen wie Alkali- und Erdalkalihydroxide, z.B. Natriumhydroxid, Kaliumhydroxid, Calciumhydroxid, Bariumhydroxid, Alkali- oder Erdalkalicarbonate wie Natriumcarbonat, Kaliumcarbonat, Calciumcarbonat, Natriumhydrogencarbonat, Kaliumhydrogencarbonat, Amide wie Natriumamid sowie organische Basen wie tertiäre Amine, z.B. Triäthylamin, N,N-Dimethylanilin, Pyridine, Chinoline und Isochinoline. Anstelle eines der üblichen Säurebindemittel kann man bei der Umsetzung auch in vorteilhafter Weise einen Überschuß des Reaktionspartners Ammoniak, Amin oder Hydrazin verwenden.

Bei der Durchführung des erfindungsgemäßen Verfahrens setzt man auf 1 Mol des 3a,4,5,6-Tetrahydro-perimidins Formel (II) mindestens 1 Mol Ammoniak, Amin oder Hydrazin der Formel (III) und gegebenenfalls mindestens 1 Mol eines der genannten Säurebindemittel ein.

Im Verlauf der erfindungsgemäßen Umsetzung gehen die Ausgangsstoffe in der Regel ganz oder teilweise in Lösung, während die Endprodukte auskristallisieren. Die Abscheidung der Endprodukte kann durch Abkühlen und/oder durch Zugabe von Fällungsmitteln wie Wasser, niedere aliphatische Äther wie Diäthyläther oder Dibutyläther oder niedere aliphatische Kohlenwasserstoffe wie Petroläther oder Leichtbenzin beschleunigt werden.

Von besonderem Interesse sind 2-Amino-3a,4,5,6-tetrahydro-perimidin-Derivate der Formel (I) in welcher die Gruppe

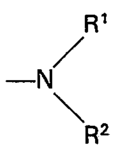

für einen gegebenenfalls substituierten Piperazino-Rest steht, wobei als Substituenten dieses Restes an den C-Atomen niedere Alkylgruppen und als Substituenten an dem N-4-Atom Folgende Reste in Frage Kommen: Wasserstoff, Phenyl, gegebenenfalls substituiert durch Nitro, Fluor, Chlor oder Amino, Benzyl oder die Gruppe

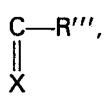

wobei X und R''' die oben angegebene Bedeutung besitzen.

Als neue Wirkstoffe seien beispielhaft genannt:

2-Amino-3a,4,5,6-tetrahydro-perimidin
2-Hydrazino-3a,4,5,6-tetrahydro-perimidin

2-Butylamino-3a,4,5,6-tetrahydro-perimidin
2-Diäthylamino-3a,4,5,6-tetrahydro-perimidin
2-Pyrrolidino-3a,4,5,6-tetrahydro-perimidin
2-Hexamethylenimino-3a,4,5,6-tetrahydro-perimidin
2-Piperazino-3a,4,5,6-tetrahydro-perimidin
2-(4-Methyl)-piperazino-3a,4,5,6-tetrahydro-perimidin
2-(4-Benzyl)-piperazino-3a,4,5,6-tetrahydro-perimidin
2-(4-Phenyl)-piperazino-3a,4,5,6-tetrahydro-perimidin
2-[4-(4-Fluorphenyl)-piperazino]-3a,4,5,6-tetrahydro-perimidin
2-[4-(3,4-Dichlorphenyl)-piperazino]-3a,4,5,6-tetrahydro-perimidin
2-[4-(4-Chlorphenyl)-piperazino]-3a,4,5,6-tetrahydro-perimidin
2-(4-Acetyl)-piperazino-3a,4,5,6-tetrahydro-perimidin
2-(4-Benzoyl)-piperazino-3a,4,5,6-tetrahydro-perimidin
2-(4-Furyl-(2)-carbonyl)-piperazino-3a,4,5,6-tetrahydro-perimidin
2-(4-Phenyl)-piperazino-7-chlor-3a,4,5,6-tetrahydro-perimidin
2-(4-Phenyl)-piperazino-9-chlor-3a,4,5,6-tetrahydro-perimidin
2-(4-Phenyl)-piperazino-7,9-dichlor-3a,4,5,6-tetrahydro-perimidin
2-(4-Phenylimidoacyl)-piperazino-3a,4,5,6-tetrahydro-perimidin
2-(4-Phenylaminocarbonyl)-piperazino-3a,4,5,6-tetrahydro-perimidin
2-(4-Phenylaminothiocarbonyl)-piperazino-3a,4,5,6-tetrahydro-perimidin
2-Phenylamino-3a,4,5,6-tetrahydro-perimidin
2-(4-Methylpiperazino)-propylamino-3a,4,5,6-tetrahydro-perimidin
2-(2-Pyridylmethyl)-amino-3a,4,5,6-tetrahydro-perimidin
2-(2-Pyridyläthyl)-amino-3a,4,5,6-tetrahydro-perimidin
2-Phenylhydrazino-3a,4,5,6-tetrahydro-perimidin
2-Dimethylhydrazino-3a,4,5,6-tetrahydro-perimidin
2-(2-Pyridyl)-hydrazino-3a,4,5,6-tetrahydro-perimidin
2-Acetylamino-3a,4,5,6-tetrahydro-perimidin

Die neuen Wirkstoffe können sowohl in den tautomeren Formeln Ia und/oder IB bzw. für den Fall, daß $R^1$ für Wasserstoff steht, auch in den entsprechenden tautomeren 2-Imino-Formen vorliegen. Darüber hinaus können sie in Form ihrer optischen Antipoden, der reinen D- bzw. L-Form, wie auch als Racetame verwendet werden.

Die neuen Verbindungen sind als Arzneimittel, insbesondere als kreislaufbeeinflussende Stoffe verwendbar.

Die erfindungsgemäßen Verbindungen haben ein breites und vielseitiges pharmakologisches Wirkungsspektrum.

Im einzelnen weisen die erfindungsgemäßen Verbindungen folgende Hauptwirkungen auf:

1. Die neuen Verbindungen senken den Blutdruck von normotonen und hypertonen Tieren und können somit als antihypertensive Mittel verwendet werden.
2. Die neuen Verbindungen bewirken an isoliert durchströmten Herzen eine deutliche und lang anhaltende Erweiterung der Coronargefäße.
3. Der Tonus der glatten Muskulator der Gefäße wird unter der Wirkung der Verbindungen stark vermindert. Diese gefäßspasmolytische Wirkung kann im gesamten Gefäßsystem stattfinden oder sich mehr oder weniger isoliert in umschriebenen Gefäßgebieten (wie z.B. in der Muskulatur, dem Zentralnervensystem u.a.) manifestieren.
4. Die Verbindungen haben muskulär-spasmolytische Wirkungen, die an der glatten Muskulatur des Magens, des Darmtraktes, des Urogenitaltraktes und des Respirationstraktes deutlich werden.

Die neuen Verbindungen sind demnach zur Vorbeugung, Besserung oder Heilung von Erkrankungen geeignet, bei denen insbesondere die oben angegebenen Effekte erwünscht sind.

Zur vorliegenden Erfindung gehören pharmazeutische Zubereitungen, die neben nichttoxischen, inerten pharmazeutisch geeigneten Trägerstoffen eine oder mehrere der erfindungsgemäßen Verbindungen oder deren Salze enthalten oder die aus einer oder mehrerer der erfindungsgemäßen Verbindungen oder deren Salzen bestehen, sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile z.B. Tabletten, Dragees, Kapseln, Pillen, Suppositorien und Ampullen vorliegen, deren Wirkstoffgehalt einen Bruchteil oder einem Vielfachen einer Einzeldosis entspricht. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nichttoxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen,

Granulate, Suppositorien, Lösungen, Suspensionen und Emulsionen genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumbicarbonat, (e) Lösungsverzögerer, z.B. Paraffin, und (f) Resorptionsbeschleuniger, z.B. quarternäre Ammoniumverbindungen, (g) Netzmittel, z.B. Cetylalkohol, Glycerinmonostearat, (h) Adsorptionsmittel, z.B. Kaolin und Bentonit, und (i) Gleitmittel, z.B. Talkum, Calcium- und Magnesiumstearat und feste Polyäthylenglykole oder Gemische der unter (a)—(i) aufgeführten Stoffe.

Die Tabletten, Dragees, Kapseln, Pillen und Granulate können mit den üblichen gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffen auch in mikroverkapselter Form vorliegen.

Suppositorien können neben dem oder den Wirkstoffen die üblichen wasserlöslichen oder wasserunlöslichen Trägerstoffe enthalten, z.B. Polyäthylenglykole, Fette, z.B. Kakaofett, und höhere Ester (z.B. $C_{14}$-Alkohol mit $C_{16}$-Fettsäure) oder Gemische dieser Stoffe.

Lösungen und Emulsionen können neben dem oder den Wirkstoffen die üblichen Trägerstoffe wie Lösungsmittel, Lösungsvermittler und Emulgator, z.B. Wasser, Äthylalkohol, Isopropylalkohol, Äthylcarbonat, Äthylacetat, Benzylalkohol, Benzylbenzoat, Propylenglykol, 1,3-Butylenglykol, Dimethylformamid, Öle, insbesondere Baumwollsaatöl, Erdnußöl, Maiskimöl, Olivenöl, Ricinusöl und Sesamöl, Glycerin, Glycerinformal, Tetrahydrofurfurylalkohol, Polyäthylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe, wie flüssige Verdünnungsmittel, z.B. Wasser, Äthylalkohol, Propylenglykol, Suspendiermittel, z.B. äthoxylierte Isostearylalkohole, Polyoxyäthylensorbit- und -sorbitanester, mikrokristalline Cellulose, Aluminiummetahydroxid, Betonit, Agar-Agar und Tragant oder Gemische dieser Stoffe enthalten.

Die genannten Formulierungsformen können auch Färbemittel, Konservierungsstoffe sowie geruchs- und geschmacksverbessernde Zusätze, z.B. Pfefferminzöl und Eukalyptusöl und Süßmittel, z.B. Saccharin enthalten.

Die therapeutisch wirksamen Verbindungen sollen in den oben aufgeführten pharmazeutischen Zubereitungen vorzugsweise in eine Konzentration von etwa 0,1 bis 99,5, vorzugsweise von etwa 0,5 bis 95 Gew.-% der Gesamtmischung vorhanden sein.

Die oben aufgeführten pharmazeutischen Zubereitungen können außer Verbindungen der Formel (I) und/oder deren Salzen auch andere pharmazeutische Wirkstoffe enthalten.

Die Herstellung der oben aufgeführten pharmazeutischen Zubereitungen erfolgt in übliches Weise nach bekannten Methoden, z.B. durch Mischen des oder der Wirkstoffe mit dem oder den Trägerstoffen.

Zur vorliegenden Erfindung gehören auch die Verwendung der Verbindungen der Formel (I) und/oder deren Salzen sowie von pharmazeutischen Zubereitungen, die eine oder mehrere Verbindungen der Formel (I) und/oder deren Salze enthalten, in der Human- und Veterinärmedizin zur Verhütung, Besserung und/oder Heilung der oben aufgeführten Erkrankungen.

Die Wirkstoffe oder die pharmazeutischen Zubereitungen können vorzugsweise oral, parenteral und/oder rectal, vorzugsweise oral und parenteral, insbesondere perlingual und intravenös appliziert werden.

Im allgemeinen hat es sich als vorteilhaft erwiesen, den oder die Wirkstoffe bei parenteraler (intravenöser) Applikation in Mengen von etwa 0,01 bis etwa 50, vorzugsweise von 0,05 bis 10 mg/kg Körpergewicht je 24 Stunden und bei oraler Applikation in Mengen von etwa 0,1 bis etwa 200, vorzugsweise 1 bis 50 mg:kg Körpergewicht je 24 Stunden, gegebenenfalls in Form mehrerer Einzelgaben zur Erzielung der gewünschten Ergebnisse zu verabreichen. Dine Einzelgabe enthält den oder die Wirkstoffe, vorzugsweise in Mengen von etwa 0,01 bis etwa 20, insbesondere 0,1 bis 2 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Zur vorliegenden Erfindung gehören auch solche Arzneimittel, die neben Verbindungen der Formel (I) noch weitere Wirkstoffe enthalten. Vorzugsweise seien genannt: Saluretica, Diuretika, andere kreislaufbeeinflussende Wirkstoffe, Phsychotherapeutica und Analgetika.

Herstellungsbeispiele

Beispiel 1

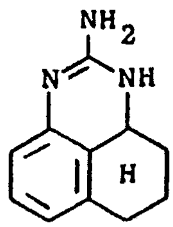

In einem Autoklaven werden 62 g (0,3 Mol) 2-Chlor-3a,4,5,6-tetrahydro-perimidin in 400 ml Äthanol und 150 ml flüssigem Ammoniak 6 Stunden auf 150° erhitzt. Der Druck steigt dabei auf max. 30 bar. Nach dem Erkalten wird das Reaktionsgemisch zur Trockene eingedampft. Man löst den Rückstand in Wasser, filtriert die Lösung über Aktivkohle und fällt das gebildete 2-Amino-3a,4,5,6-tetrahydro-perimidin durch Zugabe von wäßrigem Ammoniak. Nach dem Absaugen und Trocknen erhält man 40 g (71% d. Th.), Schmp. 230—232°.

Herstellung von 2-Chlor-3a,4,5,6-tetrahydro-perimidin:

a) Ein Autoklav wird mit 368 g (2 Mol) Perimidon-(2), 900 ml Tetrahydrofuran, 500 ml Wasser und 75 g Raney-Nickel beschickt. Nach Verdrängen der Luft mit Stickstoff wird das eingesetzte Gemisch mit Wasserstoff bis zu einem Druck von 100 bar beaufschlagt und unter Rühren auf 175°C erhitzt. Sobald diese Temperatur erreicht ist, wird der Wasserstoffdruck auf 150 bar erhöht und nach Maßgabe des Verbrauches während der gesamten Reaktionszeit aufrechterhalten. Nachdem die Wasserstoffaufnahme beendet ist, wird zur Vervollständigung der Umsetzung noch eine Stunde unter den vorangenannten Hydrierbedingungen weiter gerührt, anschließend auf Raumtemperatur abgekühlt und auf Normaldruck entspannt. Das Reaktionsgemisch wird mit 500 ml Dimethylformamid versetzt, auf 60 bis 75°C erwärmt und durch Filtration im warmen Zustand vom Katalysator getrennt. Die als Filtrat angefallene Reaktionslösung wird auf 0 bis 10°C abgekühlt, wonach ein Teil des Reaktionsproduktes in reiner kristalliner Form ausfällt und abgretrennt wird. Die Mutterlauge wird dann auf ein Volumen von etwa 700 ml eingedampft und der Rückstand mit 700 ml Wasser versetzt, wonach weiteres Reaktionsprodukt ausfällt. Man erhält insgesamt 342 g 2-Hydroxy-3a,4,5,6-tetrahydro-perimidin, Schmp. 230—233°C. Ausbeute: 90% der Theorie.

b) 100 g (0.53 Mol) 2-Hydroxy-3a,4,5,6-tetrahydro-perimidin werden in 500 ml Phosphoroxichlorid 5 Stunden auf 110° (Rückflußtemperatur) erhitzt. Unter kräftiger HCl-Entwicklung entsteht gegen Ende der Reaktionszeit eine klare Lösung, die auf ca. 60° abgekühlt wird. Nach Zugabe von 1000 ml Tetrachlorkohlenstoff rührt man weiter unter Eiskühlung, wobei das Hydrochlorid des gebildeten 2-Chlor-3a,4,5,6-tetrahydro-perimidin (Fp. 182—185°) auskristallisiert. Nach dem Absaugen und Waschen mit Tetrachlorkohlenstoff rührt man das Produkt in eine Mischung von überschüssigem Ammoniak und Eis ein und läßt unter gelegentlichem Umrühren und weiterer Eiszugabe ca. 1 Stunde lang die freie Base auskristallisieren. Diese wird abgesaugt und mit Wasser gewaschen. Ausbeute: 94 g (86% d.Th.), Schmp. 144—146°.

Beispiel 2

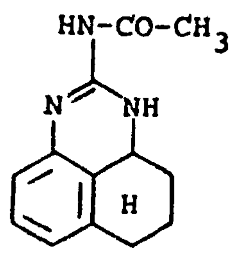

x HCl

3,7 g (0,02 Mol) des Reaktionsproduktes aus Beispiel 1 werden in 10 ml Eisessig mit 4,1 g (0,04 Mol) Essigsäureanhydrid 8 Stunden auf 115°C (Rückflußtemperatur) erhitzt. Nach dem Erkalten dampft man die Lösung zur Trockene ein und verrührt den Rückstand mit 10%iger äthanolischer Salzsäure. Nach dem Absaugen und Waschen mit Äthanol erhält man 1,9 g (38% d.Th.) 2-Acetylamino-3a,4,5,6-tetrahydro-perimidinhydrochlorid, Schmp. 229—230°C.

Beispiel 3

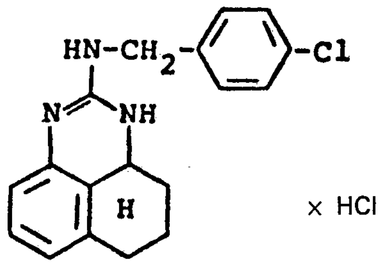

x HCl

10,3 g (0,05 Mol) 2-Chlor-3a,4,5,6-tetrahydro-perimidin und 7,1 g (0,05 Mol) 4-Chlor-benzylamin werden in 100 ml Äthanol 1 Stunde auf 80°C erhitzt. Nach dem Erkalten werden die ausgefallenen Kristalle abgesaugt und durch Lösen in heißem Äthanol und Fällen mit Wasser gereinigt. Man erhält 2-(4-Chlorbenzylamino)-3a,4,5,6-tetrahydro-perimidin-hydrochlorid. Ausbeute: 10 g (57,5% d.Th.), Schmp. 257—258°C.

Beispiel 4

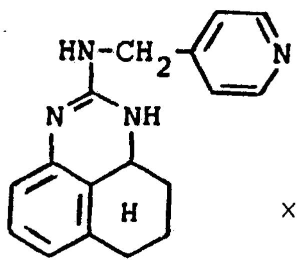

x HCl

Aus 10,3 g (0,05 Mol) 2-Chlor-3a,4,5,6-tetrahydro-perimidin und 5,9 g (0,055 Mol) 4-Aminomethylpyridin in 50 ml Äthanol erhält man analog Beispiel 3 in 2 Stunden bei 80°C 8,5 g (54% d.Th.) 2-(4-Pyridylmethylamino)-3a,4,5,6-tetrahydro-perimidinhydrochlorid, Schmp. 228—230°C (aus Äthanol).

Beispiel 5

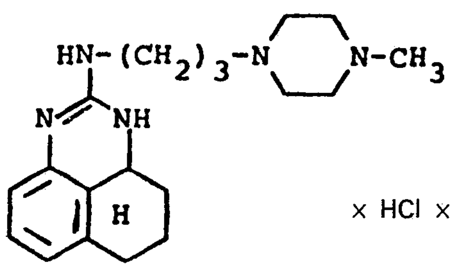

x HCl x $H_2O$

10,3 g (0,05 Mol) 2-Chlor-3a,4,5,6-tetrahydro-perimidin und 8,6 g (0,055 Mol) N-Methyl-N'-aminopropyl-piperazin werden in 50 ml Aceton 2 Stunden auf 55°C erhitzt. Nach dem Erkalten fügt man 50 ml Äther zu und stellt das Reaktionsgemisch 24 Stunden in einen Kühlschrank. Man saugt die ausgefallenen Kristalle ab und wäscht sie mit Äther nach. Dabei werden 10,6 (55,6% d.Th.) 2-(4-Methylpiperazinopropylamino)-3a,4,5,6-tetrahydro-perimidinhydrochlorid-hydrat erhalten. Schmp. 155—157°C.

Beispiel 6

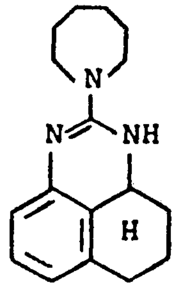

x 2 HCl x 1/2 $H_2O$

20,6 g (0,1 Mol) 2-Chlor-3a,4,5,6-tetrahydro-perimidin und 10,9 g (0,11 Mol) Hexamethylenimin werden in 100 ml Äthanol 1,5 Stunden auf 80°C erhitzt. Nach dem Abkühlen fügt man 100 ml Petroläther zu und isoliert die gebildeten Kristalle durch Absaugen. Zur Reinigung löst man das Produkt in warmem Wasser, filtriert über Aktivkohle und versetzt das Filtrat mit überschüssiger Salzsäure. Dabei kristallisiert 2-Hexamethylenamino-3a,4,5,6-tetrahydro-perimidin-dihydrochlorid-halbydrat aus. Ausbeute: 12,2 g (35% d.Th.), Schmp. 277—280°C.

Beispiel 7

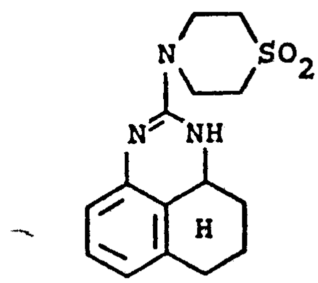

× $H_2O$

Aus 10,3 g (0,05 Mol) 2-Chlor-3a,4,5,6-tetrahydro-perimidin und 7,4 g (0.055 Mol) Tetrahydro-1,4-thiazin-1,1-dioxid in 50 ml Dioxan erhält man analog Beispiel 3 in 2 Stunden bei 100°C 15,3 g 2-(Tetrahydro-1,4-thiazino-1,1-dioxid)-3a,4,5,6-tetrahydro-perimidin-hydrochlorid. Beim Anschlämmen mit verdünnter Natronlauge bildet sich das Hydrat der freien Base. Ausbeute: 11,4 g (71% d.Th.), Schmp. 228—230°C.

Beispiel 8

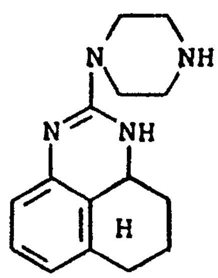

× 1/2 $H_2O$

103 g (0,5 Mol) 2-Chlor-3a,4,5,6-tetrahydro-perimidin und 485 g (2,5 Mol) Piperazinhexahydrat werden in 1000 ml Isopropanol 1 Stunde auf 80°C (Rückflußtemperatur) erhitzt. Nach dem Erkalten dampft man das Reaktionsgemisch am Rotavapor zur Trockene ein. Der Rückstand wird mit verdünnter Natronlauge versetzt und das ausgeschiedene Öl in Methylenchlorid aufgenommen. Nach dem Trocknen über Natriumsulfat, Eindampfen der Lösung und Verrühren des Rückstandes mit Essigsäureäthylester kristallisiert 2-Piperazino-3a,4,5,6-tetrahydro-perimidin-halbhydrat aus. Ausbeute: 85 g (66% d.Th.), Schmp. 170—172°C.

Beispiel 9

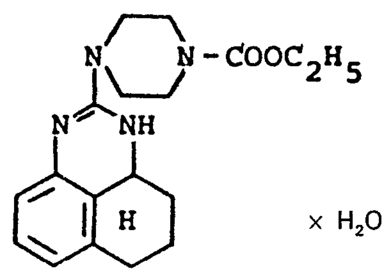

× $H_2O$

Zu 12,8 g (0,05 Mol) des Reaktionsproduktes aus Beispiel 8 in 50 ml Äthanol tropft man unter Rühren 8,1 g (0,05 Mol) Pyrokohlensäurediäthylester. Unter lebhafter $CO_2$-Entwicklung steigt die Temperatur auf 35°C. Nach 7 Stunden dampft man das Reaktionsgemisch am Rotavapor ein, versetzt den Rückstand mit Wasser und isoliert das gebildete 2-(4-Äthoxycarbonylpiperazino)-3a,4,5,6-tetrahydro-perimidin-hydrat durch Absaugen. Ausbeute: 13,8 g (80% d.Th.), Schmp. 137—139°C (aus Äthanol).

Beispiel 10

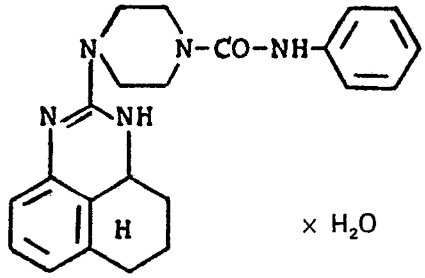

$\times H_2O$

Zu 12,8 g (0,05 Mol) des Reaktionsproduktes aus Beispiel 8 in 50 ml Pyridin tropft man bei 0—5°C (Eiskühlung) 6,5 g (0,055 Mol) Phenylisocyanat. Man rührt 10 Stunden bei Raumtemperatur, verdampft das Lösungsmittel am Rotavapor, versetzt den Rückstand mit Wasser und isoliert nach 12-stündigem Stehen 2-(4-Phenylaminocarbonylpiperazino)-3a,4,5,6-tetrahydro-perimidin-hydrat durch Absaugen. Durch Waschen mit Aceton erhält man das Präparat in analysenreiner Form. Ausbeute: 6,5 g (33% d.Th.), Schmp. 202—205°C.

Beispiel 11

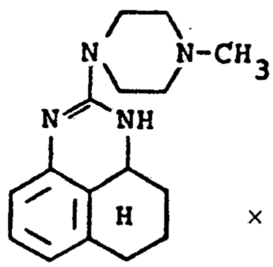

$\times 2HCl \times H_2O$

Analog Beispiel 3 erhält man aus 20,6 g (0,1 Mol) 2-Chlor-3a,4,5,6-tetrahydro-perimidin und 11 g (0,11 Mol) N-Methyl-piperazin in 100 ml Methylisobutylketon in 30 Minuten bei 100°C 25,5 g (71% d.Th.) 2-(4-Methylpiperazino)-3a,4,5,6-tetrahydro-perimidin-dihydrochlorid-hydrat. Schmp. 322—324°C.

Beispiel 12

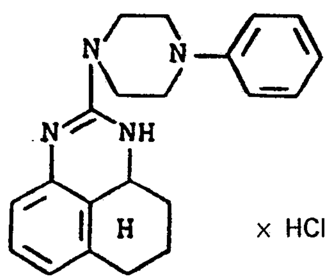

× HCl

Analog Beispiel 3 erhält man aus 30,9 g (0,15 Mol) 2-Chlor-3a,4,5,6-tetrahydro-perimidin und 24,3 g (0,15 Mol) N-Phenylpiperazin in 100 ml Tetrahydrofuran in 1 Stunde bei 65°C 42 g (76% d.Th.) 2-(4-Phenylpiperazino)-3a,4,5,6-tetrahydro-perimidin-hydrochlorid. Schmp. 296—297°C.

Beispiel 13

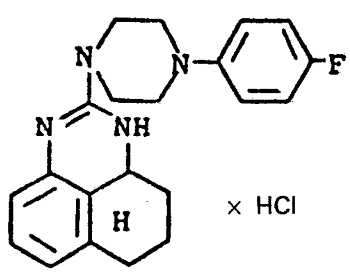

× HCl

Analog Beispiel 3 erhält man aus 9,36 g (0,045 Mol) 2-Chlor-3a,4,5,6-tetrahydro-permidin und 9 g (0,05 Mol) N-(4-Fluorphenyl)-piperazin in 50 ml Äthanol in 1 Stunde bei 80°C 14,3 g (82% d.Th.) 2-[4-(4-Fluorphenyl)-piperazino]-3a,4,5,6-tetrahydro-perimidin-hydrochlorid. Schmp. 290—293°C.

Beispiel 14

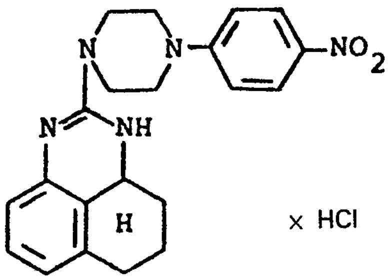

x HCl

Analog Beispiel 3 erhält man aus 59,8 g (0,29 Mol) 2-Chlor-3a,4,5,6-tetrahydro-perimidin und 60,4 g (0,32 Mol) N-(4-Nitrophenyl)-piperazin in 500 ml Methanol in 2 Stunden bei 60°C 102 g (85% d.Th.) 2-[4-(4-Nitrophenyl)-piperazino]-3a,4,5,6-tetrahydro-perimidin-hydrochlorid. Schmp. 345°C Zers.

Beispiel 15

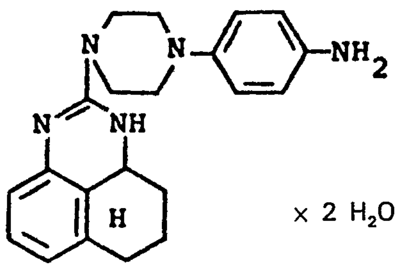

x 2 $H_2O$

75 g (0,18 Mol) des Reaktionsproduktes aus Beispiel 14 werden in 350 ml Dimethylformamid in Gegenwart von 15 g Raney-Nickel in 3 Stunden bei 50°C hydriert. Das Reaktionsgemisch versetzt man mit 100 ml 10%iger Salzsäure, saugt warm vom Katalysator ab und dampft am Rotavapor zur Trockene ein. Durch Anschlämmen des Rückstandes mit verdünntem wäßrigem Ammoniak, Absaugen und Waschen mit Wasser erhält man 2-[4-(4-Aminophenyl)-piperazino]-3a,4,5,6-tetrahydro-perimidin-dihydrat. Ausbeute: 39,7 g (57,6% d.Th.), Schmp. 305°C.

Beispiel 16

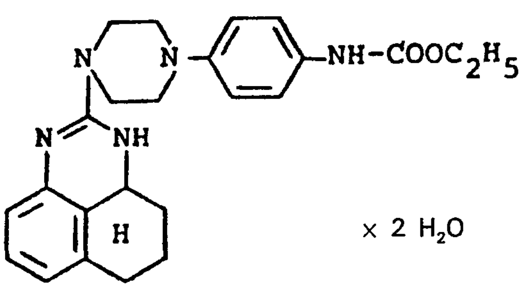

x 2 $H_2O$

Analog Beispiel 9 erhält man aus 7,7 g (0,02 Mol) des Reaktionsproduktes aus Beispiel 15 und 6,48 (0,04 Mol) Pyrokohlensäurediäthylester in 6 Stunden bei 80°C 8,6 g (94,5% d.Th.) 2-[4-(4-Äthoxycarbonylaminophenyl)-piperazino]-3a,4,5,6-tetrahydro-perimidin-dihydrat. Schmp. 280—282°C.

Beispiel 17

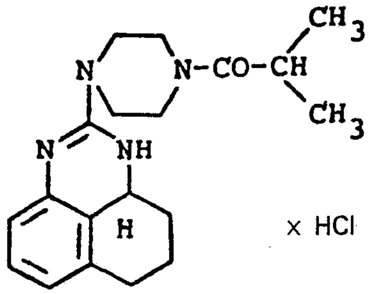

x HCl

Analog Beispiel 6 erhält man aus 10,3 g (0,05 Mol) 2-Chlor-3a,4,5,6-tetrahydro-perimidin und 8,58 g (0,055 Mol) N-Isobutyrylpiperazin in 50 ml Äthanol in 1 Stunde bei 80°C 15 g (83% d.Th.) 2-(4-Isobutyrylpiperazino)-3a,4,5,6-tetrahydro-perimidin-hydrochlorid. Schmp. 295—297°C.

Beispiel 18

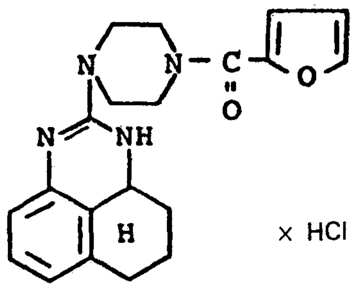

x HCl

Analog Beispiel 3 erhält man aus 10,3 g (0,05 Mol) 2-Chlor-3a,4,5,6-tetrahydro-perimidin und 9,9 g (0,055 Mol) N-Furyl-(2)-carbonyl-piperazin in 80 ml n-Propanol in 30 Minuten bei 90°C 17,5 g (90,7% d.Th.) 2-(4-Furyl-(2)-carbonyl-piperazino)-3a,4,5,6-tetrahydro-perimidin-hydrochlorid. Schmp. 299—300°C.

Beispiel 19

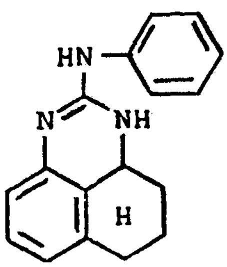

10,3 g (0,05 Mol) 2-Chlor-3a,4,5,6-tetrahydro-perimidin werden unter Rühren in 15 ml Anilin eingetragen. Nach Abklingen der stark exothermen Reaktion (Temperatur steigt bis 90°C an) versetzt man den Ansatz mit Wasser, isoliert die ausgefallenen Kristalle durch Absaugen und wäscht sie mehrfach mit verdünnter wäßriger Ammoniaklösung. Man erhält 2-Phenylamino-3a,4,5,6-tetrahydro-perimidin. Ausbeute: 9,5 g (72% d.Th.), Schmp. 233—235°C.

Beispiel 20

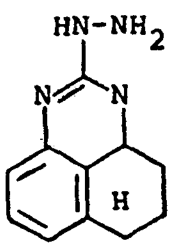

61,8 g (0,3 Mol) 2-Chlor-3a,4,5,6-tetrahydro-perimidin werden mit 19,2 g (0,6 Mol) wasserfreiem Hydrazin in 250 ml Äthanol 4 Stunden auf 80°C erhitzt. Nach dem Erkalten gibt man 250 ml Petroläther zu und isoliert die gebildeten Kristalle durch Absaugen. Man erhält 2-Hydrazino-3a,4,5,6-tetrahydro-perimidin. Ausbeute: 54 g (89% d.Th.), Schmp. 191—193°C.

Beispiel 21

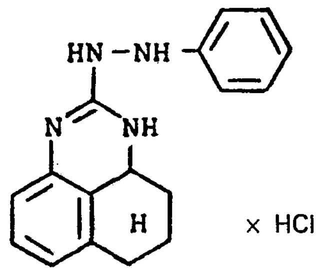

× HCl

Analog Beispiel 6 erhält man aus 10,3 g (0,05 Mol) 2-Chlor-3a,4,5,6-tetrahydro-perimidin und 8,1 g (0,075 Mol) Phenylhydrazin in 100 ml Äthanol in 1 Stunde bei 80°C nach dem Fällen mit Petroläther 11 g (70% d.Th.) 2-Phenylhydrazino-3a,4,5,6-tetrahydro-perimidin-hydrochlorid. Schmp. 243—245°C.

Beispiel 22

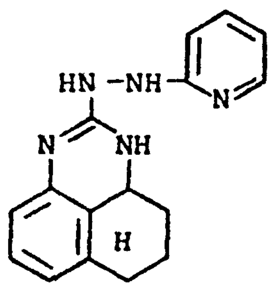

20,6 g (0,1 Mol) 2-Chlor-3a,4,5,6-tetrahydro-perimidin und 12 g (0,11 Mol) 2-Hydrazinopyridin werden in 100 ml Methanol 1 Stunde auf 60°C erhitzt. Nach dem Erkalten läßt man Eiswasser zulaufen und fällt das gebildete 2-(2-Pyridylhydrazino)-3a,4,5,6-tetrahydro-perimidin durch verdünnte Natronlauge aus. Nach dem Absaugen und Waschen mit Wasser erhält man ein Rohprodukt, das durch Waschen mit Essigsäureäthylester analysenrein anfällt. Ausbeute: 22,5 g (81% d.Th.), Schmp. 205—207°C.

Beispiel 23

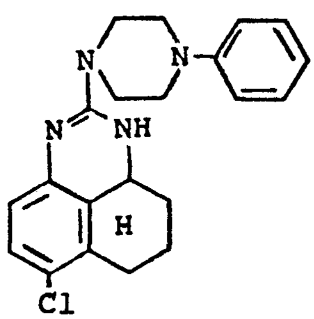

Analog Beispiel 3 erhält man aus 12,1 g (0,05 Mol) 2,7-Dichlor-3a,4,5,6-tetrahydro-perimidin (Schmp. 190—192°C) und 8,1 g (0,05 Mol) N-Phenyl-piperazin in 50 ml Äthynol in 1 Stunde bei 80°C 17,5 g 2 - (4 - Phenyl) - piperazino - 7 - chlor - 3a,4,5,6 - tetrahydro - perimidin - hydrochlorid, Schmp. 290—291°C. Durch Lösen in einer Mischung aus Aceton und wäßrigem Ammoniak und Fällen mit Wasser erhält man daraus 15,3 g (83% d.Th.) der Base, Schmp. 178—179°C.

Beispiel 24

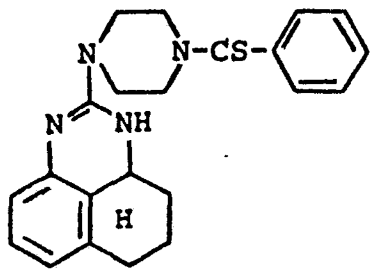

8,6 g (0,033 Mol) des Reaktionsproduktes aus Beispiel 8 und 6 g Dithiobenzoesäuremethylester werden in 15 ml 1,2-Dichlorbenzol 1 Stunde auf 150°C erhitzt, wobei sich Methylmercaptan entwickelt. Nach ca. 30 Minuten entsteht ein Niederschlag, der nach dem Abkühlen auf 25°C unter Zusatz von Äther abgesaugt wird. Man erhält 7 g (56% d.Th.) 2-(4-Thiobenzoyl)-piperazino-3a,4,5,6-tetrahydro-perimidin, Schmp. 230—232°C.

Beispiel 25

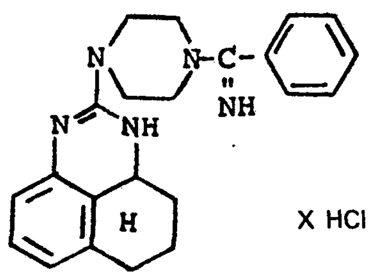

X HCl

Man erhitzt ein Gemisch von 6,4 g (0,025 Mol) des Reaktionsproduktes aus Beispiel 8, 15 ml Xylol und 4,5 g Iminobenzoesäuremethylester-hydrochlorid 6 Stunden auf 145°C, kühlt ab und saugt das Reaktionsprodukt unter Zusatz von Äther ab. Dabei entstehen 7 g (66% d.Th.) 2-(4-Phenylimidoacyl)-piperazino-3a,4,5,6-tetrahydro-perimidin-hydrochlorid, Schmp. 230°C (Zers).

Beispiel 26

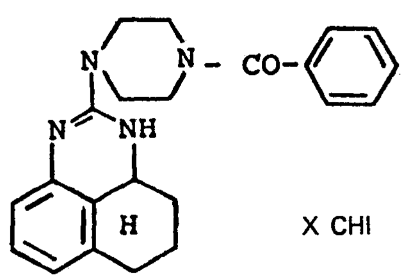

X CHl

Zu 12,8 g (0,05 Mol) des Reaktionsproduktes aus Beispiel 8 in 150 ml Pyridin tropft man bei 0—5°C (Eiskühlung) 7,7 g (0,055 Mol) Benzoylchlorid. Man läßt den Ansatz auf Raumtemperatur kommen und erhitzt anschließend 5 Stunden auf 60°C. Das Lösungsmittel wird im Vacuum abdestilliert und der Rückstand mit Wasser versetzt, wobei 2-(4-Benzoyl)-piperazino-3a,4,5,6-tetrahydroperimidin-hydrochlorid auskristallisiert. Nach dem Absaugen und Waschen mit Wasser erhält man 10 g (50% d.Th.), Schmp. 295—297°C.

Beispiel 27

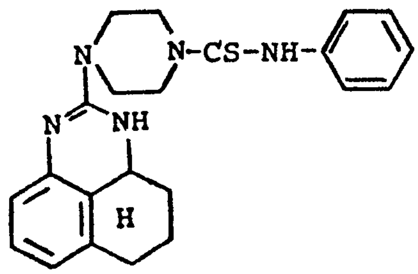

Aus 12,8 g (0,05 Mol) des Reaktionsproduktes aus Beispiel 8 und 7,5 g (0,055 Mol) Phenylsenföl in 100 ml Pyridin erhält man analog Beispiel 10 bei 20—25°C in 10 Stunden 16,1 g (82% d.Th.) 2-(4-Phenylaminothiocarbonyl)-piperazino-3a,4,5,6-tetrahydro-perimidin, Schmp. 236—238°C.

**Patentansprüche**

1. 2-Amino-3a,4,5,6-tetrahydro-perimidin-Derivate der tautomeren Formel I

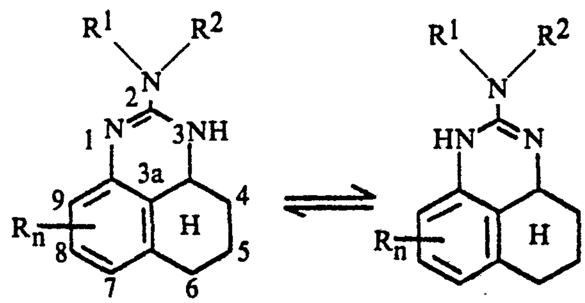

(Ia) (I) (Ib)

in welcher

R für Wasserstoff, Chlor oder Brom steht,

n für 1 oder 2 steht,

$R^1$ für Wasserstoff, Alkyl mit 1 bis 4 Kohlenstoffatomen oder Phenyl steht, und

$R^2$ für Wasserstoff, Phenyl, Acyl mit 2 bis 4 Kohlenstoffatomen, Alkyl mit 1 bis 4 Kohlenstoffatomen, wobei der Alkylrest gegebenenfalls substituiert ist durch Phenyl, Chlorphenyl, Pyridyl oder Piperazino, wobei der Piperazinorest gegebenenfalls substituiert ist durch Alkyl mit 1 bis 4 Kohlenstoffatomen, oder für Amino, Phenylamino, Pyridylamino, Mono- und Dialkylamino mit jeweils 1 bis 4 Kohlenstoffatomen in den Alkylgruppen steht oder

$R^1$ und $R^2$ gemeinsam für einen Alkylenrest mit bis zu 6 Kohlenstoffatomen stehen, wobei dieser Alkylenrest gegebenenfalls durch Sauerstoff, den $SO_2$- oder den $NR''$-Rest unterbrochen ist, wobei

$R''$ für Wasserstoff, Alkyl mit 1 bis 4 Kohlenstoffatomen, Benzyl, Phenyl — gegebenenfalls substituiert durch Halogen, Nitro, Amino oder Alkoxycarbonylamino mit 1 bis 4 Kohlenstoffatomen in der Alkoxygruppe — oder für die Gruppe

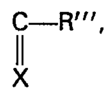

wobei

X Sauerstoff, Schwefel oder NH bedeutet, und

$R'''$ für Alkyl mit 1 bis 4 Kohlenstoffatomen, Alkoxy mit 1 bis 4 Kohlenstoffatomen, Furyl, Phenyl, Benzyl, Amino, Mono- und Dialkylamino mit jeweils 1 bis 4 Kohlenstoffatomen im Alkylrest, Phenylamino oder Pyridylamino steht,

sowie ihre pharmakologisch verträglichen Salze.

2. 2-Amino-3a,4,5,6-tetrahydroperimidin-Derivate gemäß der Formel (I) in Anspruch 1, in welcher die Gruppe

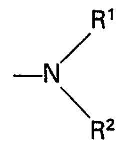

für einen gegebenenfalls substituierten Piperazinorest steht, wobei als Substituenten dieses Restes an den C-Atomen niedere Alkylgruppen und als Substituenten an dem N-4-Atom folgende Reste in Frage kommen: Wasserstoff, Phenyl, gegebenenfalls substituiert durch Nitro, Fluor, Chlor oder Amino, Benzyl oder die Gruppe

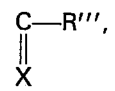

wobei X und R''' die im Anspruch 1 angegebene Bedeutung besitzen.

3. 2-(4-Phenylpiperazino)-3a,4,5,6-tetrahydro-perimidin.

4. Verfahren zur Herstellung von 2-Amino-3a,4,5,6-tetrahydro-perimidin-Derivaten der Formel (I)

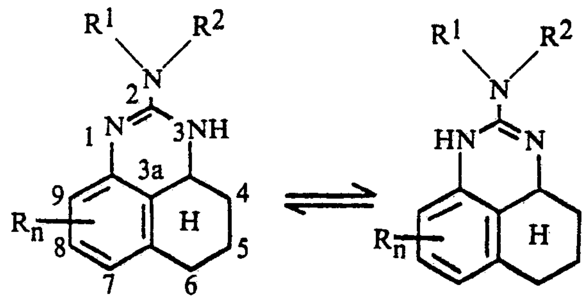

(Ia) (I) (Ib)

in welcher

R für Wasserstoff, Chlor oder Brom steht,

n für 1 oder 2 steht,

$R^1$ für Wasserstoff, Alkyl mit 1 bis 4 Kohlenstoffatomen oder Phenyl steht, und

$R^2$ für Wasserstoff, Phenyl, Acyl mit 2 bis 4 Kohlenstoffatomen, Alkyl mit 1 bis 4 Kohlenstoffatomen, wobei der Alkylrest gegebenenfalls substituiert ist durch Phenyl, Chlorphenyl, Pyridyl oder Piperazino, wobei der Piperazinorest gegebenenfalls substituiert ist durch Alkyl mit 1 bis 4 Kohlenstoffatomen, oder für Amino, Phenylamino, Pyridylamino, Monoalkylamino oder Dialkylamino mit jeweils 1 bis 4 Kohlenstoffatomen in den Alkylgruppen steht, oder

$R^1$ und $R^2$ gemeinsam für einen Alkylenrest mit bis zu 6 Kohlenstoffatomen stehen, wobei dieser Alkylenrest gegebenenfalls durch Sauerstoff, den $SO_2$- oder den NR''-Rest unterbrochen ist, wobei

R'' für Wasserstoff, Alkyl mit 1 bis 4 Kohlenstoffatomen, Benzyl, Phenyl — gegebenenfalls substituiert durch Halogen, Nitro, Amino oder Alkoxycarbonylamino mit 1 bis 4 Kohlenstoffatomen in der Alkoxygruppe — oder für die Gruppe

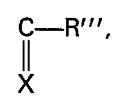

wobei

X Sauerstoff, Schwefel oder NH bedeutet, und

R''' für Alkyl mit 1 bis 4 Kohlenstoffatomen, Alkoxy mit 1 bis 4 Kohlenstoffatomen, Furyl, Phenyl, Benzyl, Amino, Monoalkylamino, Dialkylamino mit jeweils 1 bis 4 Kohlenstoffatomen im Alkylrest, Phenylamino oder Pyridylamino steht,

dadurch gekennzeichnet, daß man 3a,4,5,6-Tetrahydro-perimidine der tautomeren Formel (II)

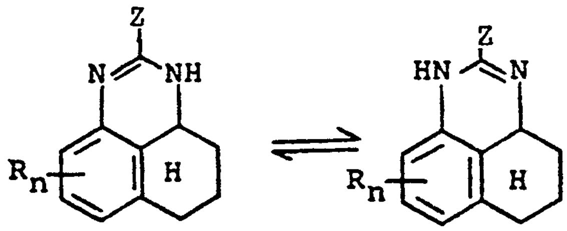

(IIa) (IIb)

in welcher

R die oben angegebene Bedeutung hat und

Z für einen nucleophil substituierbare Abgangsgruppe wie Halogen, Alkoxy, Aryloxy, Mercapto, Alkylmercapto, Arylmercapto, Alkylsulfonyl, Arylsulfonyl oder Sulfo steht, wobei die vorgenannten Alkyl- und Alkoxyreste jeweils 1 bis 8 Kohlenstoffatome enthalten und Aryl für Phenyl oder Naphthyl steht,

mit Aminen der Formel

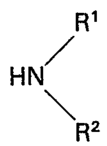

(III)

in welcher

$R^1$ und $R^2$ die oben angegebene Bedeutung haben, oder mit Salzen dieser Amine mit anorganischen oder organischen Säuren oder Basen in gegenwart von inerten organischen Lösungsvermittlern und gegebenenfalls in Gegenwart von Säurebindern bei Temperaturen zwischen 20°C und 250°C umsetzt, und gegebenenfalls Verbindungen der Formel (I), welche noch eine NH- oder $NH_2$-Gruppe besitzen, anschließend in an sich bekannter Weise acyliert.

5. Verfahren gemäß Anspruch 4, dadurch gekennzeichnet, daß man als Acylierungsmittel Halogenide von Carbonsäuren oder Kohlensäureesterhalogenide, Carbonsäureanhydride, Pyrokohlensäureester, Isocyanate oder Senföle einsetzt.

6. Arzneimittel, enthaltend mindestens ein 2-Amino-3a,4,5,6-tetrahydro-perimidin gemäß Anspruch 1.

7. Verfahren zur Herstellung von Arzneimitteln, dadurch gekennzeichnet, daß man 2-Amino-3a,4,5,6-tetrahydroperimidine gemäß Anspruch 1, gegebenenfalls unter Zusatz von inerten, pharmazeutisch unbedenklichen Hilfs- und Trägerstoffen, in eine geeignete Applikationsform überführt.

8. 2-Amino-3a,4,5,6-tetrahydro-perimidin-Derivate der allgemeinen Formel I gemäß Anspruch 1 zur Verwendung bei der Bekämpfung von Erkrankungen.

9. 2-Amino-3a,4,5,6-tetrahydro-perimidin-Derivate der allgemeinen Formel I gemäß Anspruch 1 zur Verwendung bei der Bekämpfung von Kreislauferkrankungen.

**Revendications**

1. Des dérivés de 2-amino-3a,4,5,6-tétrahydro-périmidine de formule tautomère I:

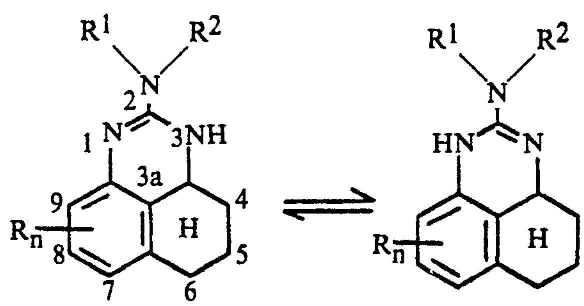

(Ia) (I) (Ib)

dans laquelle:

R est de l'hydrogène, du chlore ou du brome,

*n* est égal à 1 ou 2,

$R^1$ est de l'hydrogène, un reste alkyle ayant 1 à 4 atomes de carbone ou un reste phényle,

$R^2$ est de l'hydrogène, un reste phényle, acyle ayant 2 à 4 atomes de carbone, alkyle ayant 1 à 4 atomes de carbone, le reste alkyle étant éventuellement substitué par un reste phényle, chlorophényle, pyridyle ou pipérazino, le reste pipérazino étant éventuellement substitué par un reste alkyle ayant 1 à 4 atomes de carbone, ou un groupe amino, phénylamino, pyridylamino, mono-et dialkylamino avec à chaque cas 1 à 4 atomes de carbone dans les groupes alkyle, ou

$R^1$ et $R^2$ forment ensemble un reste alkylène ayant jusqu'à 6 atomes de carbone, ce reste alkylène étant éventuellement interrompu par de l'oxygène, le reste $SO_2$ ou le reste NR'', où

R'' est de l'hydrogène, un reste alkyle ayant 1 à 4 atomes de carbone, un reste benzyle, phényle — éventuellement substitué par un halogène, un radical nitro, amino ou alkoxycarbonylamino ayant 1 à 4 atomes de carbone dans le groupe alkoxy — ou le groupe

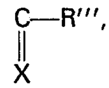

dans lequel

X est de l'oxygène, du soufre ou un groupe NH, et R''' est un reste alkyle ayant 1 à 4 atomes de carbone, alkoxy ayant 1 à 4 atomes de carbone, furyle, phényle, benzyle, amino, mono- et dialkylamino ayant dans chaque cas 1 à 4 atomes de carbone dans le reste alkyle, un reste phénylamino ou un reste pyridylamino,

ainsi que leurs sels acceptables du point de vue pharmacologique.

2. Des dérivés de 2-amino-3a,4,5,6-tétrahydro-périmidine de formule (I) suivant la revendication 1, dans laquelle le groupe

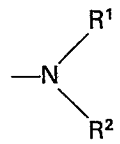

est un reste pipérazino éventuellement substitué, les substituants de ce reste sur les atomes de carbone étant des groupes alkyle inférieurs et les substituants sur l'atome N-4 étant l'hydrogène, un reste phényle, portant éventuellement un substituant nitro, fluoro, chloro ou amino, un reste benzyle ou le groupe de formule

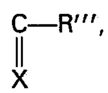

où X et R''' ont la définition indiquée dans la revendication 1.

3. La 2-(4-phénylpipérazino)-3a,4,5,6-tétrahydro-périmidine.

4. Procédé de production de dérivés de 2-amino-3a,4,5,6-tétrahydro-périmidine de formule (I):

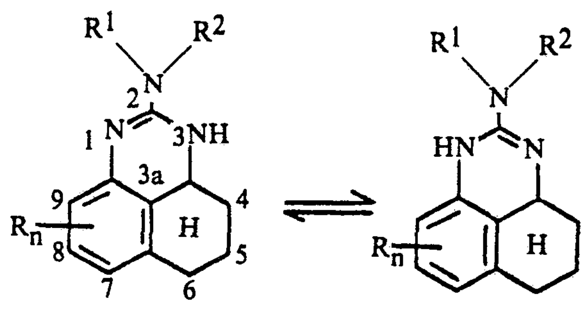

(Ia) (I) (Ib)

dans laquelle:

R est de l'hydrogène, du chlore ou du brome,

*n* est égal à 1 ou 2,

$R^1$ est de l'hydrogène, un reste alkyle ayant 1 à 4 atomes de carbone ou un reste phényle,

$R^2$ est de l'hydrogène, un reste phényle, acyle ayant 2 à 4 atomes de carbone, alkyle ayant 1 à 4

atomes de carbone, le reste alkyle étant éventuellement substitué par un reste phényle, chlorophényle, pyridyle ou pipérazino, le reste pipérazino étant éventuellement substitué par un reste alkyle ayant 1 à 4 atomes de carbone, ou un groupe amino, phénylamino, pyridylamino, mono- et dialkylamino avec à chaque cas 1 à 4 atomes de carbone dans les groupes alkyle, ou

$R^1$ et $R^2$ forment ensemble un reste alkylène ayant jusqu'à 6 atomes de carbone, ce reste alkylène étant éventuellement interrompu par de l'oxygène, le reste $SO_2$ ou le reste NR'', où

R'' est de l'hydrogène, un reste alkyle ayant 1 à 4 atomes de carbone, un reste benzyle, phényle — éventuellement substitué par un halogène, un radical nitro, amino ou alkoxycarbonylamino ayant 1 à 4 atomes de carbone dans le groupe alkoxy — ou le groupe

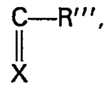

dans lequel

X est de l'oxygène, du soufre ou un groupe NH, et R''' est un reste alkyle ayant 1 à 4 atomes de carbone, alkoxy ayant 1 à 4 atomes de carbone, furyle, phényle, benzyle, amino, mono- et dialkylamino ayant dans chaque cas 1 à 4 atomes de carbone dans le reste alkyle, un reste phénylamino ou un reste pyridylamino,

caractérisé en ce qu'on fait réagir des 3a,4,5,6-tétrahydro-périmidines de la formule tautomère (II)

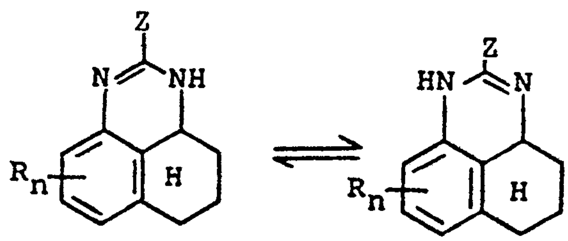

(IIa) (IIb)

dans laquelle:

R a la définition indiquée ci-dessus et

Z est un groupe partant susceptible de substitution nucléophile tel qu'un halogène, un groupe alkoxy, aryloxy, mercapto, alkylmercapto, arylmercapto, alkylsulfonyle, arylsulfonyle ou sulfo, les restes alkyle et alkoxy mentionnés renfermant dans chaque cas 1 à 8 atomes de carbone et le reste aryle étant un reste phényle ou naphtyle,

avec des amines de formule:

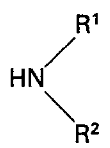

(III)

dans laquelle:

$R^1$ et $R^2$ ont la définition indiquée ci-dessus, ou avec des sels de ces amines avec des acides ou des bases inorganiques ou organiques en présence de solvants auxiliaires organiques inertes et, le cas échéant, en présence d'accepteurs d'acides à des températures de 20 à 250°C, et, le cas échéant, on acyle ensuite d'une manière connue des composés de formule (I) qui possèdent encore un groupe NH ou un groupe $NH_2$.

5. Procédé suivant la revendication 4, caractérisé en ce qu'on utilise comme agents d'acylation des halogénures d'acides carboxyliques ou des halogénures d'esters d'acides carboniques, des anhydrides d'acides carboxyliques, des esters d'acide pyrocarbonique, des isocyanates ou des sénevols.

6. Des médicaments contenant au moins une 2-amino-3a,4,5,6-tétrahydro-périmidine suivant la revendication 1.

7. Procédé de production de médicaments, caractérisé en ce qu'on convertit en une forme d'application convenable des 2-amino-3a,4,5,6-tétrahydro-périmidines suivant la revendication 1, éventuellement avec addition d'adjuvants et de supports inertes acceptables du point de vue pharmaceutique.

8. Des dérivés de 2-amino-3a,4,5,6-tétrahydro-périmidine de formule générale (I) suivant la revendication 1, destinés à être utilisés pour combattre des maladies.

9. Des dérivés de 2-amino-3a,4,5,6-tétrahydro-périmidine de formule générale (I) suivant la revendication 1, destinés à être utilisés pour combattre des maladies de la circulation.

**Claims**

1. 2-Amino-3a,4,5,6-tetrahydro-perimidine derivatives of the tautomeric formula I

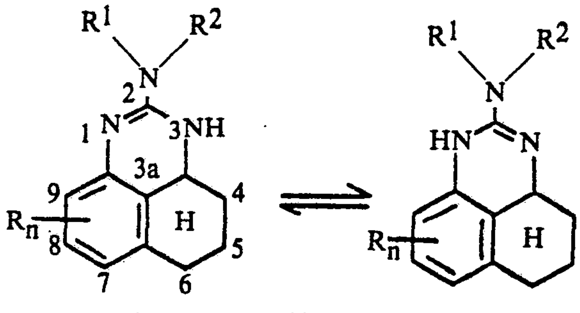

(Ia) (I) (Ib)

in which

R represents hydrogen, chlorine or bromine,

n represents 1 or 2,

$R^1$ represents hydrogen, alkyl with 1 to 4 carbon atoms or phenyl, and

$R^2$ represents hydrogen, phenyl, acyl with 2 to 4 carbon atoms, alkyl with 1 to 4 carbon atoms, the alkyl radical optionally being substituted by phenyl, chlorophenyl, pyridyl or piperazino, the piperazino radical optionally being substituted by alkyl with 1 to 4 carbon atoms, or represents amino, phenylamino, pyridylamino, mono- and di-alkylamino with in each 1 to 4 carbon atoms in the alkyl groups, or

$R^1$ and $R^2$ together represent an alkylene radical with up to 6 carbon atoms, which is optionally interrupted by oxygen, the $SO_2$ radical or the NR'' radical, wherein

R'' represents hydrogen, alkyl with 1 to 4 carbon atoms, benzyl, phenyl — optionally substituted by halogen, nitro, amino or alkoxycarbonylamino with 1 to 4 carbon atoms in the alkoxy group — or the group

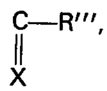

wherein

X denotes oxygen, sulphur or NH, and

R''' denotes alkyl with 1 to 4 carbon aoms, alkoxy with 1 to 4 carbon atoms, furyl, phenyl, benzyl, amino, mono- and di-alkylamino with in each case 1 to 4 carbon atoms in the alkyl radical, phenylamino or pyridylamino,

and their pharmacologically acceptable salts.

2. 2-Amino-3a,4,5,6-tetrahydro-perimidine derivatives according to formula (I) in claim 1, in which the group

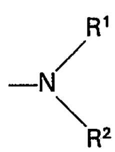

represents an optionally substituted piperazine radical, possible substituents of this radical, on the C atoms, being lower alkyl groups and possible substituents on the 4-N-atom being the following radicals: hydrogen, phenyl, optionally substituted by nitro, fluorine, chlorine or amino, benzyl or the group

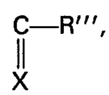

wherein X and R''' have the meaning indicated in claim 1.

3. 2-(4-Phenylpiperazino)-3a,4,5,6-tetrahydro-perimidine.

4. Process for the preparation of 2-amino-3a,4,5,6-tetrahydro-perimidine derivatives of the formula (I)

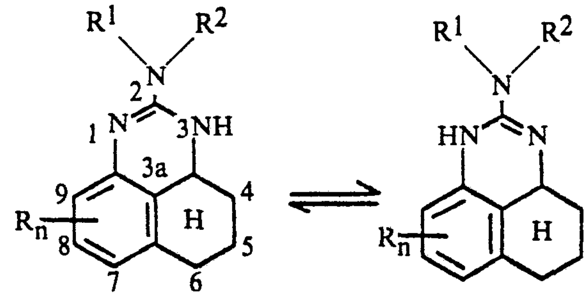

(Ia) (I) (Ib)

in which

R represents hydrogen, chlorine or bromine,

n represents 1 or 2,

$R^1$ represents hydrogen, alkyl with 1 to 4 carbon atoms or phenyl, and

$R^2$ represents hydrogen, phenyl, acyl with 2 to 4 carbon atoms, alkyl with 1 to 4 carbon atoms, the alkyl radical optionally being substituted by phenyl, chlorophenyl, pyridyl or piperazino, the piperazino radical optionally being substituted by alkyl with 1 to 4 carbon atoms, or represents amino, phenylamino, pyridylamino, mono-alkylamino or dialkylamino with in each case 1 to 4 carbon atoms in the alkyl groups, or

$R^1$ and $R^2$ together represent an alkylene radical with up to 6 carbon atoms, which is optionally interrupted by oxygen, the $SO_2$ radical or the NR'' radical,

wherein

R'' represents hydrogen, alkyl with 1 to 4 carbon atoms, benzyl, phenyl — optionally substituted by halogen, nitro, amino or alkoxycarbonylamino with 1 to 4 carbon atoms in the alkoxy group — or represents the group

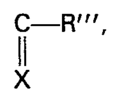

wherein

X denotes oxygen, sulphur or NH, and

R''' represents alkyl with 1 to 4 carbon atoms, alkoxy with 1 to 4 carbon atoms, furyl, phenyl, benzyl, amino, monoalkylamino, dialkylamino with in each case 1 to 4 carbon atoms in the alkyl radical, phenylamino or pyridylamino,

characterised in that 3a-4,5,6-tetrahydro-perimidines of the tautomeric formula (II)

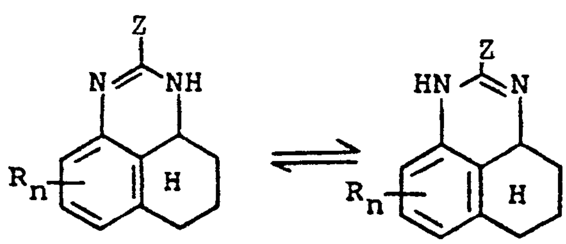

(IIa) (IIb)

in which

R has the meaning indicated above and

Z represents a leaving group which can be replaced nucleophilically, such as halogen, alkoxy, aryloxy, mercapto, alkylmercapto, arylmercapto, alkylsulphonyl, arylsulphonyl or sulpho, the aforementioned alkyl and alkoxy radicals each containing 1 to 8 carbon atoms and aryl represents phenyl or naphthyl,

are reacted with amines of the formula

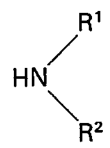

(III)

in which

$R^1$ and $R^2$ have the meaning indicated above, or with salts of these amines with inorganic or organic acids or bases in the presence of inert organic solubilising agents and if appropriate in the presence of acid-binding agents, at temperatures between 20°C and 250°C, and, if appropriate, compounds of the formula (I), which still possess a NH or $NH_2$ group are then acylated in a manner which is in itself known.

5. Process according to claim 4, characterised in that halides of carboxylic acids or carbonic acid ester halides, carboxylic acid anhydrides, pyrocarbonic acid esters, isocyanates or isothiocyanates are used as the acylating agents.

6. Medicaments containing at least one 2-amino-3a,4,5,6-tetrahydro-perimidine according to claim 1.

7. Process for the preparation of medicaments, characterised in that 2-amino-3a,4,5,6-tetrahydro-perimidines according to claim 1 are converted into a suitable form of administration, inert, pharmaceutically acceptable auxiliaries and excipients being added if appropriate.

8. 2-Amino-3a,4,5,6-tetrahydro-perimidine derivatives of the general formula I according to claim 1 for use in combating diseases.

9. 2-Amino-3a,4,5,6-tetrahydro-perimidine derivatives of the general formula I according to claim 1 for use in combating circulatory diseases.